# EUROPEAN PATENT SPECIFICATION

(11) **EP 2 095 842 B1**
(45) Date of publication and mention of the grant of the patent: **15.02.2012**
(21) Application number: 07861032.6
(22) Date of filing: 16.10.2007
(51) Int. Cl.: A61N 5/02, A61N 5/04, A61H 39/00, H01Q 21/24, A61N 5/06

(54) **DEVICE FOR OPTIMISING FUNCTIONAL STATUS OF VEGETATIVE SYSTEMS OF AN ORGANISM**
VORRICHTUNG ZUR OPTIMIERUNG DES FUNKTIONSSTATUS VEGETATIVER SYSTEME EINES ORGANISMUS
DISPOSITIF D'OPTIMISATION DE L'ETAT FONCTIONNEL DE SYSTEMES VEGETATIFS DE L'ORGANISME

(30) Priority: 07.12.2006 RU 2006144755
(43) Date of publication of application: 02.09.2009
(73) Proprietor: "TST-Group" LLC., Moscow 103383 (RU)
(72) Inventor: CHERNYAKOV, Gennady Michailovich, St. Petersburg, 197349 (RU)
(74) Representative: Reichert, Werner Franz
(86) International application number: PCT/RU2007/000569
(87) International publication number: WO 2008/069692

(56) References cited:
- RU-C2- 2 212 911
- RU-U1- 34 087
- SU-A3- 1 831 343
- SU-A3- 1 831 343
- US-A- 5 507 791
- US-B1- 6 208 903
- US-B1- 6 208 903
- JOVANOVIC-IGNJATIC Z. ET AL.: 'A review of current research in microwave resonance therapy: a novel opportunities in medical treatment' ACUPUNCT. ELECTROTHER. RES., [Online] vol. 24, no. 2, 1999, pages 105 - 125, XP008109379 Retrieved from the Internet: <URL:http://www.ncbi.nlm.nih.gov/sites/entr ez9Db=pubmed&Cmd=Search&Teim=%22Jovanovi%C4 %87-Ignjati%C4%87%20Z%22%BAuthor%D&itool=En trezSystem2PEntrezPubmedPubmedResultsPanelP ubmed_RVAbstractPlusDrugsl>

## Description

### Pertinent Art

The present invention relates in general to the art of biology, human and animal physiology, and medicine. More particularly, it is concerned with devices of optimization of man's functional condition through non-medicinal, noninvasive exposure of the human body to a flux of non-ionizing electromagnetic radiation.

### Prior Art

The use of electromagnetic energy in medicine has been known for over a century. History of the art was given in detail by S. Licht (1965), C. Susskind (1979) and A. W. Guy (1984). [S. Licht, Ed. "History of therapeutic heat," in Therapeutic Heat and Cold. New Haven, CT: Licht, 1965, pp. 196-231; C. Susskind "The 'story' ofnonionizing radiation research," Bull. N.Y. Acad. Med., vol. 55, no. 11, pp. 1152-1163, 1979; A. W. Guy "History of biological effects and medical applications of mickrowave energy" IEEE Transactions on Microwave Theory and Techniques, vol. MTT-32, no. 9, pp. 1182-1200, September 1984.] As evidenced by these authors, up to the 1970s physicians' interest in therapeutic application of electromagnetic fields and waves - over the entire range available for the purpose - was focused on the effect of volume heating of live tissues deep in the human body.

At an early stage it was believed possible to kill cancer cells in situ by local overheating, on the presumption that cancer cells are less resistant to an increase of their temperature, compared to cells of unaffected tissues of warm-blooded animals and humans. For this reason early medical microwave apparatus were designed to have a radiation power assuring heat release in the target tissues well in excess of heat removal by the tissue's blood flow. The trend was still continued after use of the electromagnetic volume heating effect was applied to different physiological responses of organisms to an artificial local overheating of their tissues.

A bulk of knowledge of these responses was obtained as a result of large-scale worldwide research aimed at development of hygienic requirements on safety of operation of microwave sources and receivers. These studies revealed that hyperthermia induced in a limited volume of live tissues can be used as a therapeutic method making it possible to attain a protective response effect, similar to those associated with local inflammations.

Because internal temperature is one of vital homeostatic parameters for warm-blooded animals a local hyperthermia is "recognized" by the organism to be a pathogenic focus and a reason to trigger a package of responses preventing propagation of morbiferous agents beyond the focus.

Application of microwave energy for inducing local hyperthermia in internal tissues and stimulating thereby protective responses of the organism has been in use for over half-century. However, the sought effect has proved difficult to control. The reason is that the package of general protective responses chosen by an organism to fight a local inflammation depends largely on how distinct the inflammation is, where it is located, and what immunity reserves are available to the organism when the inflammation starts developing. Besides, it is difficult to monitor adequately the physics of inflammation imitation, including conditions of interaction between the electromagnetic flux from the applicator and the patient's body, and to quantify the degree of hyperthermia and the volume of the hyperthermia-affected tissue. All this stems from the fact that electrodynamics, geometry and other conditions of propagation of electromagnetic waves in, and their absorption by human body's tissues have so far been studied and understood but very roughly.

A major step forward in the field of hyperthermia application was made recently by the inventors of a device which makes it possible to focus electromagnetic radiation with considerable accuracy in a target volume of human body (US Patent No. 6,208,903"). Such a device comprises not a single antenna but a plurality of antenna elements whose amplitude and phase is controlled by a software package, so as to optimize exposure parameters of the target tissue inside the body through adequate overlapping of the electromagnetic waves. Simultaneously, the same device keeps maintaining a lower radiation level in the space around the focal point. In order to adjust wave impedance of the space between the antenna array plane and surface tissues of the human body, this space is filled with a multi-layer dielectric pad that features the same passive electrodynamic parameters as the skin-fat layer. All these special features of exposing live tissues to electromagnetic waves make the above device for focusing of thermal energy inside human body substantially different from the conventional method of microwave hyperthermia of internal tissues by exposing skin to electromagnetic waves coming via air from an electromagnetic horn or another directional-type antenna.

Hyperthermia of the tissues inside the focal space occurs through absorption of electromagnetic waves at an operating frequency of approximately 2.45 GHz, which has been in use for medical applications on live tissues since the 1940s. More particularly, this frequency was used in US-made applicators by Raytheon Company and in Soviet *Luch* apparatus. A unique feature of electromagnetic waves of this frequency is that they get resonance-absorbed by water molecules; thereby the sought effect of spatial heat release gets maximized in water-rich media, such as biological tissues. It also rules out any phenomena other than heat generation because, no matter how large is the flux of microwave energy, all the absorbed part gets transformed into stochastic oscillatory motion of water molecules.

Another approach to therapeutic applications of electromagnetic waves is based on use of such energy flows that cause no local hyperthermia of tissues at all or result in a hyperthermia which is much below the level designed to kill cell elements in the hyperthermia zone or to stimulate non-specific protective forces of the organism. Such applications or exposures are generally described as 'non-thermal ', but some authors use a number of arbitrary terms ('information', 'information-wave', or 'resonant').

In the USSR, practical introduction of non-thermal electromagnetic wave therapy started in the mid-1980s under the guidance of Academician N. D. Devyatkov who had co-ordinated for close to two decades all Soviet experimental and theoretical studies in the area of interaction between millimetric radio waves and biological objects. Results of a major stage of this research effort were first reported at a special session of the USSR Academy of ScienceS⁵. Clinical practice findings were published 15 years later under separate covers.

### Disclosure of the invention

The idea behind applying millimetric electromagnetic waves of non-thermal intensity for medical purposes was a belief that they can get involved directly, i. e. without being transformed into heat, in regulatory processes of tissues, organs, physiological systems and organisms in vivo by performing a certain information function.

The idea was essentially put forward in the late 1960s by A. S. Presman⁷ who suggested a hypothetical mechanism behind observable changes in animals' typical behavior after exposure to microwaves of such a low intensity that no physically detectable traces of their absorption by live tissues could be recorded.

Presman emphasized that the term "informative action" he introduced as a synonym of "non-thermal action" should not be interpreted in the sense that there was some kind of a specific interaction between extra-weak electromagnetic waves and live organisms. Still, enthusiastic adherents of medical applications of such radiation insisted on regarding non-thermal effects as strictly specific, in the sense that the external factor, i. e. the radiation flow, is a signal-bearing carrier of a structural sign.

A typical example of this interpretation of biophysical interaction between millimetric band electromagnetic waves and live tissues is the Russian patent RU 2212911¹² claiming a "method of reflexotherapy". Its authors' attention is focused on parametric features of millimetric band waves they apply, on their presumption that it is parameters of electromagnetic waves that can assure any preset therapeutic effect, all the way down to detailed control of "enzyme formation in stomach". In order to assure a wide variance of parameters of the actuation factor, in addition to discrete frequency adjustment over a 40 to 70 GHz range, immense for millimetric band waves, and energy flux density variations over at least three orders of magnitude (from 10 µW·cm⁻² to 5 mW·cm⁻²), the authors of the patent suggested a multi-step amplitude modulation of magnetic waves and even their circular polarization with manual control of the sense of field rotation. Any specific change in radio wave amplitude has to be set by at least five parameters at the same time. For example, harmonic oscillations proper, of any frequency within the above range, fill trapeziform pulses of variable amplitude and duration; in the process, amplitude of trapeziform pulses changes from zero to a maximum which is determined by an arbitrarily chosen value of the energy flow density, while pulse spacing and duration are set unambiguously and at the same instant with the help of a special parameter and a T-mode value. The latter is made possible by the fact that the T-mode has a time dimensionality while the special parameter is a dimensionless value which determines the number of equal time microintervals making up a T-mode, each microinterval, in its turn, being quasi-randomly distributed between the duration of a trapeziform pulse and the duration of the pause that follows it and shows a zero radiation amplitude. Over the T-mode time interval the amplitude of trapeziform pulses changes from zero to a maximum and back from the maximum to zero exponentially; thus the envelope of the resulting pattern resembles a positive polarity triangle with the base lying on the X-axis. Characteristics of the rising and trailing edge exponent of the trapeziform pulse pattern are variables that are determined from a randomly-set ratio of durations of the rising and trailing parts of the pattern.

The approach seemed to promise next to boundless creative opportunities for designing all sorts of shades of meaning for the energy flow structure, which is allegedly the carrier of a specific informative principle capable to make normal the condition of some or other tissues, organs or systems of human body, even without regard for random choice between clockwise (CW) or counterclockwise (CCW) rotation of the field. However, no one has ever succeeded to implement those opportunities for a simple reason that the patent does not offer any intelligible explication about criteria that should be used to choose a specific combination of radiation parameters for a specific health condition. Instead, one is offered general speculations on 'unity of exponential regularities of natural processes', on 'biotropicity' (see Patent¹², p. 2) of CW- and CCW-polarized radiation, allegedly assured by live organisms' selective uptake of optical isomers of amino acids and sugars, on 'essential enorganic biorhythms that are characteristic of a pathology-stricken organ' (Patent¹², p. 5), and the like. By way of illustration, it is suggested by the authors of Patent¹² that value of T-mode be selected "in proportion to those essential frequencies of the organism's biorhythms that are characteristic of an organ with a pathology", as if reference were made to commonly known and generally accepted tabulated data. A special methodological feature of projects of the kind is complete disregard of topological aspects of exposure interaction; in other words, there is no practical discussion of how natural response of an organism depends on what part of its body is exposed to electromagnetic waves. It appears to be presumed that the sought therapeutic effect is *a priori* considered to be par excellence a function of exposure parameters, much like heating of internal tissues. For example, with not a word said how interaction between millimetric electromagnetic waves and "the gastric tract" is to be organized, with this kind of radiation penetrating into water-rich media to a depth of less than 1 mm, the authors of the Patent¹² insist that "exposure of the gastric tract to CW-rotating electromagnetic waves stimulates generation of the pepsin enzyme", while "with CCW rotation, generation of pepsin is slowed down" (op. cit., p. 5).

An alternative interpretation of 'informative effect' of non-thermal electromagnetic waves on live objects is based on an assumption of a distinct resonance dependence between the radiation frequency and the resulting content of live objects' response, in other words, on the belief that a radiation of a certain frequency could "imitate" some kinds of "internal communication and control signals (information signals) of the organism" ⁸.

The suggested curative factor to be used was not any radio-frequency electromagnetic waves, which might justly fall under Presman's definition of "informative exposure", but millimetric band waves alone⁸. They were distinguished for therapeutic use as that part of the spectrum which is virtually non-existent in the Earth-reaching cosmic rays but matches frequency-wise the intrinsic frequency of the rotary motion of water molecules, abundant in biological tissues. Millimetric waves of certain frequencies, such as 42.254 GHz and 53.604 GHz, were declared to be universal carriers of internal communication signals, "common for all living substances". This assumption served the base for development of curative radiation sources using those universal frequencies, including "Yav" and "Elektronika KVCh".

Actually near-resonance responses to application of non-thermal millimetric band waves can be observed with sufficient accuracy on elementary biological models, such as hemoglobin molecules, and then only after thorough dehydration⁹. Millimetric band waves cannot initiate any specific events in live tissues that were apriori predetermined by absorption of waves' energy, similar to absorption of light quanta by eye's photoreceptors, they are always associated with changes in a package of some or other interdependent vital functions. This is testified to by observation of parallel changes in bioelectrical, metabolic and biomechanical indicators of the condition of model biological objects¹⁰.

As shown by results of clinical practice, success of therapeutic application of non-thermal millimetric band waves stems less from their frequency than from methodical details of exposure. Key factors of importance are as follows: selection of a target point (or a system of points) on the body; selection of the time of day for an exposure session; selection of exposure duration for the selected target point (selection of sequence and duration of exposures for each target point if there are more than one). For instance, application of physically identical millimetric band waves to points on the central line of abdomen in two target areas - just below the tip of the metasternum or above the top edge of the pubic articulation - can give favourable results in accelerated healing of gastric ulcer or elimination of cervical erosion, respectively. In either case success depends largely on how close the selected skin areas are to biologically active points (BAP), i. e. acupuncture loci that are recommended for insertion of the tips of needles into the skin for the purpose of treating respective disorders.

Hence what is believed to be an external effect of controlling a specific process in the organism, such as the process of regeneration of the mucous coat of stomach or cervix, is in fact largely predetermined by internal features of the target object. By the 1990s the view that therapeutic application of millimetric electromagnetic waves methodically overlaps principles of traditional Oriental medicine, particularly those of chen-tzu therapy, became fairly widespread. True, mechanisms of interaction between microwaves and BAP structures are still interpreted in the terminology of resonance processes allegedly taking place in a frequency range which coincides with that of millimetric band waves⁴.

In practical terms, drawing analogies between BAP actuation by traditional acupuncture methods and by application of non-thermal millimetric band waves is a major step forward in development on non-medicinal treatment. An indisputable advantage of the latter approach is its noninvasive character eliminating risks of infection that can be carried by body-puncturing needles.

Deplorably, mastering of new medicine-free therapeutic techniques by practitioners is hindered by an insistent emphasis on hypothetical resonance processes that allegedly assure an essentially 'informative' interaction between millimetric electromagnetic waves and the live matter, specifically the BAP structures. This accent on 'unique' properties of millimetric waves blurs and dilutes understanding of the fact that the content of curative effects is much more the result of what is anticipated from addressing this or that BAP than whatever attainable through wave parameter settings. An unavoidable sequence of this approach is a dangerous oversimplification of basic acupuncture traditions of accurate dosage of external impact that is applied to this or that BAP. For example, present-day recommendations on exposure of BAP to electromagnetic waves so far fail to indicate if the exposed active point will be in the aroused or sedated state at the end of a session limited by the exposure time alone, even though it is precisely the balance of arousal and sedation in different points that determines the treatment tactics of chen-tzu therapy.

Of as great importance is the fact that a physician who generally has only one electromagnetic applicator available can treat only one point at a time having to rely on his/her own abilities in determining a treatment sequence.

Another negative sequence of the overemphasis on the hypothesis of allegedly unique capabilities of millimetric band waves as 'messengers' to biological objects is that research efforts get distracted from electromagnetic waves of different wave bands, which, however, may be quite valuable for therapeutic treatment of human body areas larger than BAPs (e.g., Head's lines or zones).

Taking into account a BAP's small linear dimensions and area (within a few square centimeters), millimetric electromagnetic waves are technically the most adequate exposure range, a beam of such waves being easily concentrated on any BAP, with next to no overlapping of non-target-tissues, without any technical additions to the open waveguide. Accurate focusing is assured simply by keeping the antenna's exit section at a certain distance from the target skin area. Longer radiowaves, if used as the exposure factor, would call for extra appliances. A common device "for informative wave therapy" is described in the Russian patent RU 2156626. To make its emitter applicable for exposing BAPs to radiowaves of a fairly wide frequency spectrum, it is suggested to make it of a dielectric "in the form of wideband filter" so that the free end of the radiating dielectric rod be cone-shaped. This design is claimed to be essential for a geometrical agreement between the cross-section area of the beam reaching the target skin area and the natural linear dimensions of "active zones", as the author of this invention calls acupuncture points. However, with target skin areas as small as a few dozens of square centimeters and with the wavelength commensurable with linear dimensions of the target area, it would be wise to use longer-wave emissions.

To expand the application area of non-thermal or "informative" electromagnetic treatment methods, it was suggested in 1991 to use low-intensity centimetric band waves for exposing biologically active areas, in particular, Head's lines. Again in 1991, a patent was issued for an embodiment of a method of optimization of biological object's functional condition by a flux of low-intensity centimetric band waves (Russian Federation Patent Nº 1831343 dated 17.10.91, IPC: A61N 5/02, 5/04; A01G 7/04, 7/00, A01C).

It is proposed in the above patent¹⁴ that, with a wideband spiral antenna used, the microwave frequency be chosen with account for linear dimensions of the target zone (the point to be exposed to electromagnetic waves), so that, at the distance from the antenna face to the origin of the wave zone, the area of the beam's central lobe be 80 to 100% of the target body surface. At the same time it is suggested that the selected application frequency be oscillated 0 to 10% by a quasi-stochastic signal varying from 20 Hz to 50 kHz, very much like the sweep of a millimetric carrier frequency within a few per cent of its central value. The choice of a spiral antenna assures a relative ease of microwave frequency control and a fairly narrow directional pattern of electromagnetic waves.

The treatment procedure consisted in exposing an open skin area within the biologically active target zone to a flux of centimetric band waves of non-thermal intensity coming at a right angle from the face of a spiral antenna. The distance between the antenna's face section and the exposure surface had to be large enough for formation of a wave front and was set before the treatment session by moving the antenna and the patient's body about each other. A special scale bar was used to check that the distance between the antenna and the exposure surface was sufficient. The session duration was set by an electronic timer.

Practical use of non-thermal centimetric band waves has proved their zonal application to have a fairly good therapeutic effect in asthenic condition treatment after severe diseases, multimodality weight-reducing therapy, treatment of chronic nonspecific pulmonary, cardiovascular and some other diseases.

However, a number of disadvantages became obvious as well, of which the most important ones were a possibility of an erroneous choice of carrier frequency by the physician and difficulties of aiming a spiral antenna's electromagnetic waves at the target area. Errors in frequency selection led to difficulties in comparative analysis of the application results, which, in their turn, resulted in controversy about approaches to therapy of this or that disease. The situation was further aggravated by the fact that use of a spiral antenna called for keeping unchanged a preset mutual arrangement of the antenna face and the target area throughout the session. An involuntary movement of the patient's body, if not immobilized in the needed position, changed the distance between the antenna and the body leading to a change in the energy flux density, which is known to vary in inverse proportion to the square of the distance from the source to the exposed surface. Another inconvenience of a spiral antenna is that a few per cent of its total electromagnetic radiation is directed sideways, at a right angle to the main lengthwise beam along the spiral's axis, which may become an exposure hazard for those near the patient during the session.

The objective of the method suggested herein is to enhance the therapeutic efficiency of applying centimetric band electromagnetic waves to biologically active zones of the human body. Said method is not a part of the invention.

It is offered to raise the efficiency through exposure of biologically active zones of the human body to an electromagnetic field of a centimetric range:
- at a wavelength equivalent to the exposure zone;
- at a constant average density of the energy flux throughout the session;
- by amplitude modification of the exposure signal by a signal varying quasi-randomly over a 20 Hz to 50 kHz range;
- by circular polarization of the emission;
- as well as by prevention of exposure to electromagnetic fields of any part of the body other than the target area.

It has been proved by practice that any disease is cured best by a special apparatus designed and manufactured with allowance for peculiarities of the zone or area to be treated. When a single apparatus is used for treatment of a totality of diseases at any exposure areas different physicians may choose different radiation frequencies, which complicates comparison of treatment results and distorts analysis. Thus abandonment of an all-purpose frequency-adjustable electromagnetic wave applicator contributes to a better efficiency of the zonal exposure method. Application efficiency is further improved by maintaining the energy flux density unchanged throughout a treatment session and putting this vital parameter beyond the reach of chance.

It is further proposed to broaden capabilities of electromagnetic therapy through amplitude modulation of the exposure signal by an alternating signal that varies quasi-randomly over a 20 Hz to 50 kHz range and by circular polarization featuring manual control of clock-wise or counter-clock-wise rotation of the field. The prior art device uses quasi-random frequency modulation of radiation. In the proposed apparatus emission's amplitude varies with time quasi-randomly; in addition, linear polarization is replaced with circular polarization and the sense of rotation of the field is set at will. Circular polarization makes it possible to bring the applicator closer to the target surface and to maintain their mutual position unchanged throughout the session, thereby making unnecessary checks of the distance between the applicator and the target object and thus simplifying the treatment procedure, as described below:

The applicator is brought in contact with the target area of the body via a pad transparent to centimetric electromagnetic waves. Treatment emission is started by pushing an appropriate key on the front panel. A preset duration of the session is monitored automatically. Emission generation is stopped automatically when the preset time ends, and the end of the session is announced by an audible signal.

The proposed method can be executed by the device described below. Said method, however, is described for illustrative purposes only.

The prototype closest to the one disclosed here is the one covered by Russian Patent¹⁵. The prototype embodies a method of exposure of biologically active zones of the human skin to a non-thermal electromagnetic field of a centimetric wave band. It comprises a microwave oscillator, a modulator in the form of a pseudorandom noise impulse generator, and a planar antenna with a beam device for determining the optimum distance between the antenna plane and the target surface, all these units being series-connected. The beam indicator device of the prototype is essentially made up of two directed light sources mounted on the antenna body at an angle to each other in a manner that their visible beams cross in the zone which is optimum for the target area.

The prototype uses a printed-circuit phase array planar antenna, rather than a circular antenna used in Patent¹⁴. The device described in Patent¹¹ also provides for application of an 'antenna array'; there, however, unlike the components of the disclosed phase array, each element is an independent antenna with a wave amplitude and phase control channel of its own. The fact that each array element launches waves, rather than alternating electromagnetic fields, is essential for calculation of, and provision for, the sought effect of their superposition in a given point. To assure constant spatial coordinates of each wave source, all antenna elements are rigidly arranged in a multiple-cell array for launching a plurality of waves with a varying total directivity diagram. As for the device disclosed in Patent¹⁴, it launches not a plurality but a single electromagnetic wave, with the directivity diagram remaining strictly constant. Its planar antenna rules out emission in any direction other than along the perpendicular to the antenna plane, in other words, from the antenna to the target area. On the whole, however, the device disclosed in Russian Federation Patent Nº 1831343 is not free from defects described above.

A high frequency signal from the oscillator of this device comes to each radiating element of the planar antenna in one point only; that is why the resulting radiation is plane-polarized. For this reason the target surface, which is likely to reflect some waves, should be arranged at a distance of at least about 30 cm so as to avoid a detrimental effect of reflected waves on functioning of the microwave oscillator. Thus the problem of standardization of physical conditions that determine therapeutic effects of exposure to electromagnetic waves is not optimally solved in this device. Among other things, the distance to target area is not kept fixed and unchanged but is maintained by the patient who is guided by his/her subjective visual assessment of superposition of the body's target area and the intersection point of the visible indicator beams; this disagreement can lead to less efficient therapeutic effect. Besides, an involuntary movement of the patient's body away from the pre-session position may result in an undesirable change in the energy flux density during the session.

The device disclosed herein solves the problem of enhancing efficiency of electromagnetic therapeutic procedures through assuring an appropriate control of exposure parameters.

Solution of the problem is assured by the fact that in the disclosed device, which comprises a number of series-connected units including a microwave oscillator, a modulator in the form of a pseudo-random signal generator, an antenna in the form of a planar synphased array, and a control unit connected to each of these units, the antenna assures a circular polarization, generates a centimetric range field, and is provided with a planar fixed thickness pad of a material which is transparent to fields of the operating range.

The antenna is of a stripline type, with striplines of the antenna divider arranged so as to assure circular polarization of the field and to change direction of its rotation.

Thanks to the antenna's circular polarization, operation of the microwave oscillator is substantially less affected by waves that are reflected from the exposure surface, which makes it possible to cut the distance between the antenna plane and the target surface of the body to a few centimeters without any detriment to generation of therapeutic waves or their therapeutic effect. The distance from the antenna to the exposure surface of the body is set by the thickness of a dielectric pad, to be made of a material transparent to centimetric band waves, such as cotton or animal wool.
Figures illustrating the disclosed device are as follows:
Fig. 1. Block diagram of device;
Figs. 2, 3. Radiating elements of antenna;
Fig. 4. Layout of striplines feeding antenna's radiating elements for the case of varying sense of rotation of field;
Fig. 5. Layout of striplines feeding antenna's radiating elements for the case of unchanging sense of rotation of field;
Fig. 6. Side view of antenna showing mutual arrangement of printed circuit boards with striplines and radiating elements;
Fig. 7. Diagram showing mutual arrangement of root stripline and antenna element stripline of emission quality monitoring electronic system.

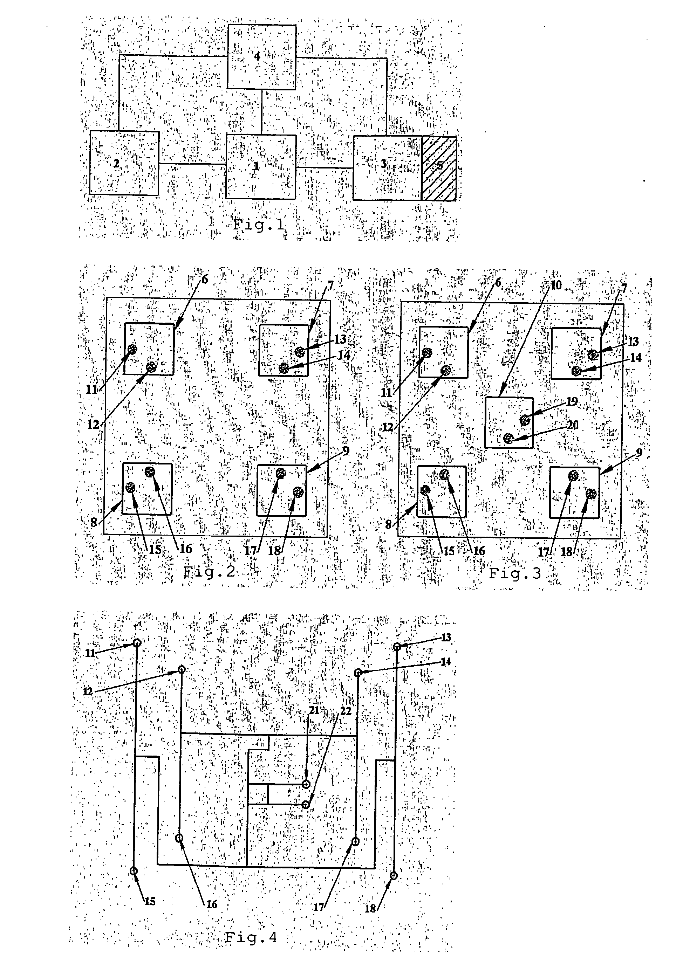

The disclosed device (Fig. 1) comprises series-connected carrier frequency oscillator 1, modulator 2 in the form of a pseudo-random signal generator, planar antenna 3, and control unit 4 connected to each of the above units. The distance between the antenna's plane and the exposure surface that assures a constant energy flux level from the carrier frequency generator is maintained by a preset thickness of the dielectric pad 5. The dielectric pad 5 is made of a material, such as cotton wool, which is transparent to fields of the operating frequency range.

The radiating antenna 3 (Figs. 2 and 3) is of a stripline printed circuit board type. Antenna 3 comprises a few (for example, four or five) radiating elements which are numbered 6 to 10, arranged as shown in Figs. 2 and 3, and jointly making up a synphased array, the outgoing beam sent at a direct angle to the antenna's surface. The stripline divider is designed to have an amplitude distribution of the field among antenna's radiating elements assuring them an excitation level that would minimize the side emission away from the main beam of the antenna. The basic configuration of the divider's striplines is found conventionally, with account taken of the fundamental frequency of emission.

Each radiating element 6 to 10 of the antenna 3 is connected to a stripline which brings the high frequency signal from the microwave oscillator to the pairs 11, 12; 13, 14; ...; 19, 20, respectively (Fig. 3). Both CW and CCW circular polarization of the field is made possible by configuration of antenna elements and by printed circuit layout of the stripline divider. The apparatus design permits alternative embodiments of the device featuring either switchable or fixed sense of the field's rotation and polarization.

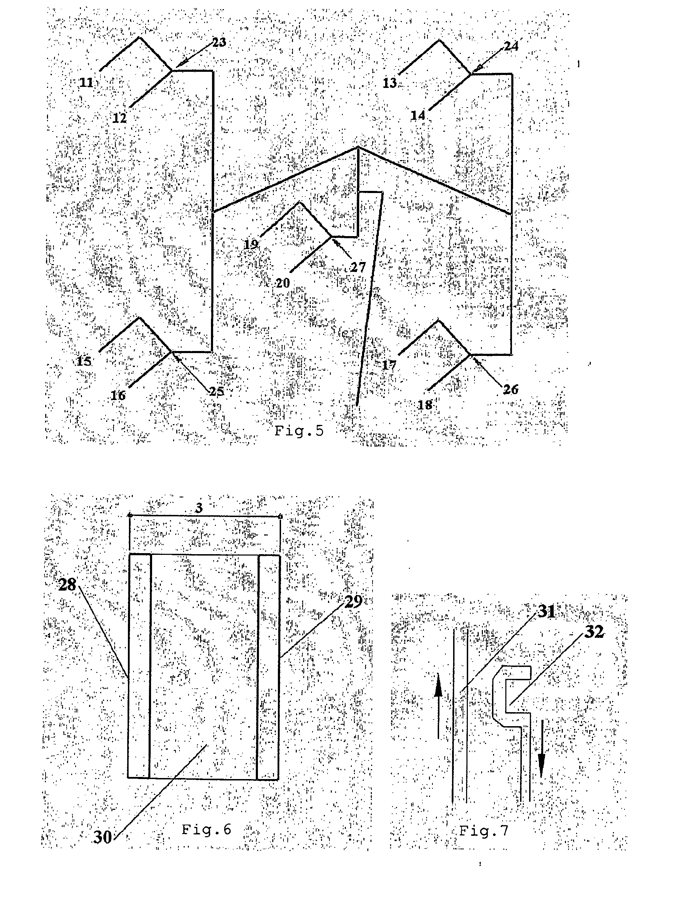

The switchable sense-of-rotation feature is provided by bringing HF excitation separately to each point of the pairs 11, 12; 13, 14 etc. of individual radiating elements 6 to 9 of the antenna 3, as shown schematically in Fig. 4 where HF signal feeding points 21 and 22 correspond to the CW and CCW rotation of the field, respectively. The fixed sense-of-rotation feature can be provided by branching a single stripline which brings a HF signal to an individual radiation element of the antenna, with arms of striplines that feed the HF signal from individual branching points 23 to 26 to the pairs 11, 12; 13, 14 etc. of individual radiating points 6 to 10 of the antenna 3 differing in length so that points of each pair be excited with a preset phase shift (Fig. 5).

Regardless of a stripline arrangement alternative, the antenna 3 consists of the p.c. board 28 with radiating elements; the p.c. board 29 with components of the stripline divider that feeds the microwave signal from the oscillator to antenna's radiating elements; and a dielectric pad 30 whose thickness must be at least 1/20^{th} of the operating wave length (Fig. 6).

In order to monitor continuously compliance of emission's quality with specifications, part of energy of signals coming from the microwave oscillator to antenna's radiating elements is picked up at the root stripline 31, which is directly connected to the microwave oscillator, see Fig. 7, and fed to the control unit via the stripline antenna element 32, a separate entity on the stripline p.c. board (not shown in Figs. 4 and 5). Arrows along the stripline 31 and the stripline branch from the antenna element 32 show the flow of signals from the microwave oscillator and to the control unit, respectively.

A method of therapeutic use of non-thermal centimetric-band electromagnetic fields can be illustrated by an example of optimization of the respiratory system when adversely affected by asthmatic symptoms and other morbidity factors, such as allergic responses, consequences of infectious diseases of bronchi and upper airways, etc., through dosed exposure of the chest-side skin projection of respiratory tracts to centimetric radio waves.

More specifically, the application part of the body is a triangle-shaped area of cutaneous covering, with two apexes resting on the middle of the collar bones and the bottom apex, at the xiphoid cartilage. Taking into account the averaged area of the central part of the triangle, the device used to apply the above therapeutic procedure generates waves at a frequency of about 4.1 GHz with a wave length of some 7.3 cm. The carrier-frequency emission is amplitude-modified from zero to the maximum by a 20 Hz to 50 kHz quasi-stochastic varying signal. Amplitude modulation of the carrier frequency may be also set within 0.1% of its basic value. Circular polarization of emission is employed. The device uses a planar antenna.

Density of the energy flux is 80 to 100 µW·cm⁻². A dielectric pad between the antenna and the exposure surface of the body is made of clean cotton wool in the form of a 6.5 to 7 cm thick pillow in a cotton pillow slip.

Stages of a therapeutic session are as follows: put the device to the wait state by pressing the appropriate key on the control panel; press the appropriate key to set duration of the session; place the pillow pad on the body area to be exposed; put the device on the pad, the radiation side down, and press it to the pad slightly; start the session by pressing the appropriate key. The end of the session is announced by an audible signal. Likewise, an audible signal, along with an announcement on the digital session time display, signals any kind of fault condition of the apparatus. th The patient controls the field's sense of rotation guided by a feeling of slight warmth in the target area, which is a sign of toward effect of the exposure. The field's sense of rotation is reversed by the patient when she/he feels a chill, pricking or another near-indefinable sensation.

Clinical trials of the method and apparatus were carried out in three clinics, two in St. Petersburg and one in Moscow. The trials involved adult patients (age group of 23 to 67 years) and children aged 2.5 to 17. Studied in both groups was applicability of the method and device for therapy of asthmatic symptoms during exacerbation periods of chronic bronchial asthma. Studies of all the cases were carried out against the background of medicinal therapy, with the diagnosis of "a severe and medium-severe course of disease".

The method of applying centimetric-band non-thermal electromagnetic waves with the help of the disclosed device was shown by the studies to be a fairly efficient non-medicinal therapy for treatment of bronchial asthma, which can be used without any limitations for curing both adults and children.

Application of the disclosed device - in strict compliance with instructions - does not result in any untoward effects; far from causing a discomfort, the treatment procedures are taken with pleasure by both adults and children.

It has been found that use of the disclosed device for treatment of bronchial asthma exacerbations in inpatients and outpatients alike contributes to a faster pulmonary functional recovery, thereby making it possible to reduce basic medicinal therapy, among other things, to lower doses of systemic glucocorticoids. In particular, with day- and night-time asthmatic fits occurring much less frequently and often disappearing on a fifth or sixth day of application of the device, a majority of adult patients need twice as less fast-acting β2-agonists. A positive attitude of the totality of patients to the treatment with the help of the disclosed device stems from relief of coughing attacks, less frequent asthmatic fits, an easier expectoration, and a subjective sensation of smoother breathing. After a longer treatment of two weeks and more users of the device express their positive emotions about a smaller scale of medicinal therapy, particularly about a reduction in use of steroidal agents. Objective studies that were carried out during the clinical tests have demonstrated that application of the disclosed device within three days leads to noticeably less intensive dry rales, a smoother breathing, and improved functional indicators of patency of airways. Starting from the fifth day of treatment with the help of the disclosed device, the special bronchodilator test turned negative.

Children's response to the curing effect of the device is much more impressive than that of adults. Treatment sessions are taken by them "with pleasure". Subjective sensations of "smooth breathing" are reported in some 70% of cases during the very first session. Treatment of exacerbations of medium-severe and even severe bronchial asthma in children very often stops asthmatic attacks on the very first day, never failing to yield such a positive result beyond a third day. Some 60% of the young patients stop using fast-acting β2-agonists on a second or third day of treatment by the disclosed device, which has been never recorded with application of medicinal therapy alone. Easier expectoration is the most welcome response because clearing the phlegm from bronchi gives children more freedom for normal physical activities.

According to findings of objective instrument-aided tests, application of the disclosed device contributes to nearly twice as fast disappearance of dry rales and a general improvement of patency of airways. The latter convalescence, noticeably better than the one attainable with medicinal therapy alone, is reliably established by instrumental monitoring of the respiratory function. On the whole, therapeutic procedures using the disclosed apparatus cut the treatment time more than twice, compared to times recorded in long-term observations. For instance, while conventional treatment of bronchial asthma cuts exacerbations short within seven to ten days, application of the disclosed apparatus results in a remission effect on a third or fourth day from the start of treatment.

### References

1. S. Licht, Editor. History of therapeutic heat, in Therapeutic Heat and Cold. New Haven, CT, 1965, pp. 196-231.
2. C. Susskind. The 'story' of nonionizing radiation research. Bull. N.Y. Acad. Med., Vol. 55, No. 11, pp. 1152-1163, 1979;
3. A. W. Guy. History of biological effects and medical applications of mickrowave energy. IEEE Transactions on Microwave Theory and Techniques, Vol. MTT-32, No. 9, pp. 1182-1200, September 1984.
4. Zlata Jovanovic-Ignjatic, Dejan Rakovic. Microwave resonant therapy: Novel opportunities in medical treatment. Acup. & Electro-Therap. Res., The Int.J. , 1999, Vol. 24, No. 2, pp. 105-125.
5. Uspekhi fizicheskikh nauk, 1973, Vol. 110, No. 3, pp. 452-469. [in Russian].
6. Millimetric Waves in Medicine and Biology. Moscow, 1989. Edited by N. D. Devyatkov, 307 p. [in Russian].
7. A. S. Presman. Electromagnetic Fields and Wildlife. Moscow, Nauka Publishers, 1968, 285 p. [in Russian].
8. N. D. Devyatkov, M. B. Golant. On the mechanism of the effect of non-thermal millimetric electromagnetic waves on vital functions of organisms. In: Effects of Application of Non-thermal Millimetric Waves to Biological Objects. Edited by N. D. Devyatkov. Moscow, 1983, pp. 18-33 [in Russian].
9. N. P. Didenko, V. I. Zelentsov, M. V. Falkovich, N. P. Fedorov. On resonance response of hemoglobin molecules versus millimetric radiation power. In: Millimetric Waves in Medicine and Biology. Moscow, 1989. Edited by N. D. Devyatkov, pp. 227-235 [in Russian].
10. G. M. Chernyakov, V. L. Korochkin, A. P. Babenko, E. V. Bigday. Responses of complex biological systems to application of low-intensity millimetric waves. In: Millimetric Waves in Medicine and Biology. Moscow, 1989. Edited by N. D. Devyatkov (Editor), pp. 140-167 [in Russian].
11. US Patent No. 6,208,903.
12. Russian Federation Patent Nº 2212911.
13. Russian Federation Patent Nº 2156626.
14. Russian Federation Patent Nº 1831343 dated 17.10.91, IPC: A61N 5/02, 5/04; A01G 7/04, 7/00, A01C 1/00.
15. Russian Federation Useful Model Patent Nº 340871 dated 30.07.2003.

## Claims

1. An apparatus for optimizing a functional condition of a human organism's vegetative system, comprising a series-connected set of a microwave oscillator (1), a modulator (2) in the form of a pseudo-random impulse signal generator, a radiating antenna (3), and a control unit (4) connected to each of the above, units (1, 2, 3), **characterized in that** the antenna (3) is arranged for radiating a centrimetric wave band electromagnetic field of non-thermal intensity, has the form of a planar co-phasal array, and is of a centimetric band circular-polarization type with a plane-wise fixed-thickness dielectric pad (5) of a material which is transparent to fields of the operating frequency range.

2. An apparatus as claimed in Claim 1, **characterized in that** the pad (5) is of a material which is transparent to electromagnetic fields of the centimetric wave band.

3. An apparatus as claimed in Claim 2, **characterized in that** the material is cotton or animal wool.

4. An apparatus as claimed in Claim 1, **characterized in that** the antenna (3) is of a stripline type.

5. An apparatus as claimed in Claim 1, **characterized in that** the antenna comprises a stripline and radiating elements (6 to 10), the radiating elements (6 to 10) being connected to the stripline.

## Patentansprüche

1. Vorrichtung zum Optimieren eines Funktionszustands des vegetativen Systems eines menschlichen Organismus, wobei die Vorrichtung einen in Reihe geschalteten Satz eines Mikrowellenoszillators (1), eines Modulators (2) in Form eines Pseudozufallsimpulssignal-Generators, einer abstrahlenden Antenne (3) und einer Steuereinheit (4), die mit jeder der obigen Einheiten (1, 2, 3) verbunden ist, umfasst, **dadurch gekennzeichnet, dass** die Antenne (3) zum Abstrahlen eines elektromagnetischen Felds im Zentimeterwellenband mit nichtthermischer Intensität ausgelegt ist, die Form eines ebenen gleichphasigen Strahlerfelds aufweist und vom Typ für das Zentimeterband und für zirkulare Polarisation mit einem dielektrischen Kissen (5) mit in Ebenenrichtung fester Dicke aus einem Material, das für Felder des Betriebsfrequenzbereichs durchlässig ist, ist.

2. Vorrichtung nach Anspruch 1, **dadurch gekennzeichnet, dass** das Kissen (5) aus einem Material besteht, das für elektromagnetische Felder des Zentimeterwellenbands durchlässig ist.

3. Vorrichtung nach Anspruch 2, **dadurch gekennzeichnet, dass** das Material Baumwolle oder Tierwolle ist.

4. Vorrichtung nach Anspruch 1, **dadurch gekennzeichnet, dass** die Antenne (3) von einem Streifenleitungstyp ist.

5. Vorrichtung nach Anspruch 1, **dadurch gekennzeichnet, dass** die Antenne eine Streifenleitung und Strahlungselemente (6 bis 10) umfasst, wobei die Strahlungselemente (6 bis 10) mit der Streifenleitung verbunden sind.

## Revendications

1. Dispositif pour l'optimisation d'un état fonctionnel du système végétatif d'un organisme humain, comprenant un ensemble en série se composant d'un oscillateur micro-ondes (1), un modulateur (2) sous la forme d'un générateur de signaux d'impulsions pseudo-aléatoires, une antenne rayonnante (3 ), et une unité de contrôle (4) raccordée à chacune des unités (1, 2, 3) ci-dessus, **caractérisé en ce que** l'antenne (3) est agencée de manière à émettre un champ électromagnétique en bande d'ondes centimétriques d'intensité non-thermique, a la forme d'une antenne multiple planaire monophasée, et est de type à polarisation circulaire en bande centimétrique avec une plaquette diélectrique (5) planaire d'épaisseur fixe fabriquée en un matériau qui est transparent aux champs de la plage de fréquences de service.

2. Dispositif selon la revendication 1, **caractérisé en ce que** la plaquette (5) est fabriquée en un matériau qui est transparent aux champs électromagnétiques de la bande d'ondes centimétriques.

3. Dispositif selon la revendication 2, **caractérisé en ce que** le matériau est le coton ou la laine animale.

4. Dispositif selon la revendication 1, **caractérisé en ce que** l'antenne (3) est de type guide d'ondes à rubans.

5. Dispositif selon la revendication 1, **caractérisé en ce que** l'antenne comprend un guide d'ondes à rubans et des éléments rayonnants (6 à 10), les éléments rayonnants (6 à 10) étant raccordés au guide d'ondes à rubans.
